## Europäisches Patentamt
## European Patent Office
## Office européen des brevets

(19)

(11) Publication number: **0 099 690**
**B1**

(12)

# EUROPEAN PATENT SPECIFICATION

(45) Date of publication of patent specification: **17.09.86**

(51) Int. Cl.⁴: **C 10 G 3/00, B 01 J 8/24, B 01 J 8/34, C 07 C 1/20**

(21) Application number: **83303870.6**

(22) Date of filing: **04.07.83**

(54) Process for the conversion of alcohols and oxygenates into hydrocarbons.

(30) Priority: **20.07.82 US 400203**

(43) Date of publication of application:
**01.02.84 Bulletin 84/05**

(45) Publication of the grant of the patent:
**17.09.86 Bulletin 86/38**

(84) Designated Contracting States:
**BE DE FR GB IT NL**

(56) References cited:
**DE-A-2 003 062**
**GB-A-1 019 236**
**US-A-3 851 405**
**US-A-4 071 573**
**US-A-4 197 418**

**CHEMIE-INGENIEUR-TECHNIK, vol. 45, no. 5, 1973, pages 307-312 B. NEUKIRCHEN et al.: "Gestaltung horizontaler Rohrbündel in Gas-Wirbelschichtreaktoren nach wärmetechnischen Gesichtspunkten"**

(73) Proprietor: **MOBIL OIL CORPORATION**
**150 East 42nd Street**
**New York New York 10017 (US)**

(72) Inventor: **Avidan, Amos Andrew**
**14 Spring Hill Road**
**Mantua New Jersey 08051 (US)**
Inventor: **Gould, Ronald Michael**
**223 Champion Way**
**Sewell New Jersey 08080 (US)**
Inventor: **Kam, Anthony Yuk-Yim**
**18 S. Birchwood Park Drive**
**Cherry Hill New Jersey 08003 (US)**

(74) Representative: **West, Alan Harry**
**Mobil Court 3 Clements Inn**
**London WC2A 2EB (GB)**

Courier Press, Leamington Spa, England.

## Description

This invention relates to the production of light olefins, hydrocarbons boiling in the gasoline and distillate boiling ranges and mixtures thereof ·from lower aliphatic alcohols, related and other oxygenates, and mixtures thereof by catalytic conversion in a horizontally baffled fluid bed of ZSM-5 type zeolite catalyst.

The growing demand for fuels and light petroleum products, including light olefin gases, and the growing shortage of crude oil supplies, have resulted in a continuing strong interest in tapping alternative raw material sources from which to obtain the desired light hydrocarbon products.

In recent years it has been discovered that alcohols and related oxygenates, which may be obtained from coal, natural gas or biomass, can be converted into more complex hydrocarbons, including gasoline boiling range hydrocarbons, by utilizing as catalysts a group of zeolites exemplified by ZSM-5 zeolite. In one commercial aspect, such a process is employed as part of the conversion of natural gas into gasoline. Natural gas is steam reformed to synthesis gas which in turn is converted into methanol. The methanol is then converted into gasoline employing Mobil Oil Corporation's MTG (methanol-to-gasoline) process. The MTG process is described in, for example, U.S. Patents, 3,894,103; 3,894,104; 3,894,107; 4,035,430 and 4,058,576. U.S. Patent 3,894,102 describes the conversion of synthesis gas into gasoline.

The conversion of lower monohydric alcohols into gasoline by the catalytic action of ZSM-5 type zeolites is highly exothermic. To control this exothermic heat, there have been employed fluidized catalyst techniques which were developed previously, principally in the petroleum industry. Fluidization methods have been usefully employed in the cracking of gas oils where a powdered catalyst was used to transfer heat, developed during the regeneration of the catalyst in a fluidized bed, to another fluidized bed of catalyst to effect the endothermic cracking of the gas oil.

The use of fluidized bed techniques not only to control the heat released in the conversion of alcohols into gasoline but also, because of the intimate contact between reactants and catalyst provided by the fluidized catalyst, to improve product selectivity and catalyst life, has also been described. However, the very nature of the fluidized catalyst bed promotes the growth of bubbles of reactant gases and the backmixing of gasiform materials, both of which are undesirable. A number of prior patents on this subject have addressed these problems in a variety of ways. Thus U.S. Patent 4,071,573 discloses the use of horizontally disposed grid means, such as wire mesh screens or heat exchanger means, in a dilute phase catalyst riser reactor to disperse reactant or product gas bubbles as the catalyst-reactant suspension passes upwardly through the riser. A plurality of vertical baffles is provided in the dense phase fluid catalyst bed described in U.S. Patent 4,197,418 and is said to restrict upflowing reactant bubble growth so as not to exceed about 150 mm and to provide substantial plug flow conditions. U.S. Patents 4,238,631 and 4,251,484 describe a dense fluidized bed reactor for methanol conversion provided with a plurality of vertical heat exchanger tubes and vertical open-ended baffle tubes which are spaced to provide a flow path having a hydraulic diameter of 100 to 200 mm when the reactants are in contact with the fluid mass of catalyst particles.

In accordance with the invention, it has been found that significant improvements can be made in the process of converting a $C_1$—$C_3$ aliphatic alcohol, particularly methanol, related oxygenates and/or oxygenates from Fischer-Tropsch synthesis into light olefins, gasoline boiling range hydrocarbons and/or distillate boiling range hydrocarbons with a ZSM-5 type catalyst in a dense fluidized bed reactor, when the reactor is provided with a plurality of horizontally disposed baffles. The baffles are tubular in form and may serve as indirect heat exchanger means to remove the exothermic heat generated in the conversion. By being located horizontally within the reactor, the baffles increase the conversion efficiency over that obtained in an unbaffled or vertically baffled dense fluidized bed of a comparable ZSM-5 type catalyst converting a comparable feed under comparable conversion conditions.

Although the use of horizontal baffles in fluidized bed reactors has previously been advocated, such baffles have always been used in conjunction with other associated equipment, for example feed distribution manifolds, vertical baffles and vertical catalyst downcomers, for providing particular baffling effects. The horizontally disposed, tubular baffles used in accordance with the present invention achieve the desired increase in conversion efficiency totally independently, thereby precluding the need to use any supplementary means to achieve an additional baffling effect.

According to the present invention, there is provided a process for converting a reactant comprising a $C_1$—$C_3$ monohydric alcohol, a related oxygenate, a Fischer-Tropsch oxygenate or a mixture of any two or more thereof, into a hydrocarbon product of higher boiling point which comprises passing a feed comprising the reactant alone or together with water upwardly through a dense fluidized bed of particles of a catalyst comprising a ZSM-5 type crystalline zeolite under exothermic conditions to convert at least 70% of the reactant into the hydrocarbon product, characterized in that the dense fluidized bed of catalyst particles contains a plurality of spaced, horizontally-disposed, tubular baffles in a plurality of spaced rows, the rows of baffles being so arranged in a staggered pattern as to provide between adjacent baffles a triangular or square configuration having a pitch of 50 to 200 mm. Preferably, the rows of horizontal tubular baffles are arranged as a series of bundles of baffles with each bundle having a vertical height of 0.3 to 1.4 m and being spaced from an adjacent bundle by from 0.75 to 3 m.

The feed to the process of the invention may include a $C_1$—$C_3$ monohydric alcohol alone or a mixture of such alcohols. The feed may also include mixtures of such an alcohol or alcohols, related oxygenates and/or oxygenates produced by Fischer-Tropsch synthesis alone, as mixtures thereof or as mixtures with water. Examples of related oxygenates are the ethers of the useful alcohols, i.e., dimethyl ether (a related oxygenate of methanol). Water may comprise up to 20 weight percent of the feed to this process where conversions of at least 95% are desired. Where partial conversion of at least 70%, is desired water may comprise up to 60% weight percent of the feed.

The $C_1$—$C_3$ monohydric alcohols that may be charged to the process include methanol, ethanol, propanol, and isopropanol. The feed may consist of a relatively pure single alcohol, or a mixture of such alcohols. In general, any mixture comprising methanol, ethanol, propanol, isopropanol or mixtures thereof and which is convertible with high exothermicity, is a suitable feed for the process. Conversions which produce more than 230 kJ/kg of total hydrocarbon product, and preferably more than 450 kJ/kg of hydrocarbon product, at conversion temperature, are considered highly exothermic for the purpose of the present invention.

The preferred charge of the present invention and the one utilized in the detailed description that follows is methanol together with up to 20 weight percent of water. Mixtures of methanol and dimethyl ether are also preferred.

In the following description, the process is described with methanol serving as the feedstream. This is done for convenience and for illustrative purposes only since it will be appreciated that the feed can comprise any of the other alcohols or oxygenates described herein either alone or as mixtures with water.

The conversion effected in the process is achieved in the presence of a catalyst comprising a crystalline zeolite that is a member of a class of zeolites known as ZSM-5 type zeolites. The ZSM-5 type zeolites used in accordance with the invention are crystalline zeolites having a silica/alumina ratio greater than 12 and a Constraint Index (C.I.) from 1 to 12. These zeolites and their use as conversion catalysts for lower aliphatic alcohols are described in U.S. Patents 3,894,106, 4,025,571, 4,058,576 and 4,148,835.

The ZSM-5 type zeolites are exemplified by ZSM-5, ZSM-11, ZSM-12, ZSM-23, ZSM-35 and ZSM-38, with ZSM-5 being particularly preferred. ZSM-5 is described in U.S. Patent 3,702,886; ZSM-11 is described in U.S. Patent 3,709,979; ZSM-12 is described in U.S. Patent 3,832,449; ZSM-23 is described in U.S. Patent 4,076,842; ZSM-35 is described in U.S. Patent 4,016,245; and ZSM-38 described in U.S. Patent 4,046,859.

Particularly preferred catalysts are those comprising ZSM-5 type zeolite of large crystal size, i.e. a crystal size of at least 1 micrometer, as described in U.S. Patents, 4,025,571 and 4,148,835.

The process of the invention is concerned with improving the conversion in a dense fluidized bed reactor when catalytically converting alcohols into higher boiling hydrocarbons, particularly methanol into gasoline boiling range hydrocarbons. A measure of this improved conversion may be made by comparing the conversion with that obtained in a comparable unbaffled fluidized bed reactor operating at complete plug flow of the reactant mixture, i.e., no backmixing. Dividing the conversion obtained in the reactor under study by that calculated if the same reactor has been operated under ideal plug flow conditions provides a percentage approach to the optimum which is designated conversion efficiency.

In the process of the invention carried out in a horizontally baffled reactor, the exothermic heat of reaction obtained when converting a methanol charge is absorbed by the catalyst and in substantial measure by the horizontal tubular baffles utilized as heat exchanger tubes for the generation of high pressure steam. Where the horizontal baffles serving as heat exchangers do not remove sufficient heat from the reactor, additional heat transfer can be obtained, for example by providing vertically disposed heat exchanger tubes between the horizontal baffle sections.

The temperature in the fluid catalyst bed is substantially uniform. Fluid bed temperatures may range from 300 to 430°C. Usually bed temperatures of 340 to 430°C are employed for producing gasoline while bed temperatures of 300 to 370°C are used when a highly olefinic product is desired. According to one aspect of the invention, the feed is brought into the bottom of the reactor riser at about 175°C where it is immediately heated to about 300°C when it contacts the hot zeolite catalyst. The feed temperature rises as it passes up the riser, exiting at about 315°C into the dense bed of catalyst which is operating at 400 to 410°C.

When methanol is used as the feed, it is preferred to achieve a methanol conversion of greater than 95% and preferably 99.5% to minimize the loss of difficultly recoverable methanol in the water formed in the process. At the operating conditions defined, including a pressure restriction of about 310 kPa at the bottom of the fluid mass of catalyst and about 270 kPa in the reactor dispersed catalyst phase and relying upon a nominal gas velocity of about 0.6 m/sec., the density of the fluid mass of catalyst in the lower portion of the reactor is about 320 to 480 kg/m$^3$. Reactor bed density depends on a number of variables including catalyst type, particle size distribution and gas velocity. When it is desired to change product selectivity to one of high olefin content as opposed to high aromatics' yield, the reaction time as a function of space velocity may be reduced by increasing the space velocity. On the other hand, aromatic formation is enhanced by a reduction in reactant space velocity to increase time of contact between methanol reactant product and catalyst. A good balance in product selectivity between olefins, aromatics and paraffins may be had by a proper selection of reaction conditions.

The baffles employed according to the invention are disposed horizontally to obtain the beneficial results described herein. Whereas vertical baffles are said to restrict the size of gaseous bubbles and to define a flow path having hydraulic diameter within a desired range, horizontal baffles do not serve these functions. It is postulated that the effectiveness of the horizontal baffles is based on the staging effect they provide in that the spacing configurations provide what essentially appears to be a multiplicity of small reaction zones within the dense fluidized catalyst bed.

Horizontal baffles in any of a variety of forms may be employed. Thus the baffles may be hollow tubes or pipes such as heat exchanger tubes. The baffles are disposed horizontally in a series of rows with the baffles in each row having a particular spaced relationship and adjacent rows also having a particular spaced relationship.

A plurality of horizontally disposed tubular baffles may, therefore, fulfill several functions. Some of them may serve as part of an indirect heat exchanger while all of them provide a baffling function. Heat exchanger tubes having an external diameter from 25 to 75 mm are most conveniently employed as the tubular baffles. The several rows of baffles may be arranged in a staggered pattern to provide a spacing configuration between adjacent tubes in a triangular or a square pattern, either of which should have a pitch from 50 to 200 mm. The spacing of the tubular baffles is dependent on the intended gas velocity in the fluidized bed as well as the properties of the catalyst. In general, the higher the gas velocity and the lower the catalyst density, the more open should be the tubular baffle spacing or pitch. For example, for a superficial gas velocity of 0.4 to 0.9 m/sec. and a catalyst solid density (weight/total volume) of 0.7—1.2 g/cc, the baffle tubes should be at least 75 mm apart (wall-to-wall) to obtain a conversion efficiency of 65 to 85%.

It has been found that the tubular baffles can usefully be arranged as bundles of tubes with each bundle disposed horizontally across the fluidized bed reactor and comprising a number of vertical rows of tubes. Where the baffles are being utilized as a heat transfer means, one or more bubbles of tubular baffles may serve as heat exchangers. An effective number of these bundles may be provided to remove the exothermic heat of reaction released within the fluidized bed reactor, thereby serving as a significant and convenient source of process steam for this unit and other processing units in the vicinity. The tubes are conveniently arranged in bundles with each bundle having a vertical height from 0.3 to 1.4 m and with each bundle separated from an adjacent bundle by a vertical distance from 0.75 to 3 m, preferably from 0.9 to 1.8 m. This vertical spacing appears to improve the conversion efficiency by providing a number of small individual reaction zones rather than a single large reaction zone. It is postulated that the improvements are obtained principally because the tubular baffle configuration significantly reduces the backmixing of the reactants and product thereby limiting undesirable side reactions or prolonged exposure to thermal cracking temperatures.

The effectiveness of a particular configuration of baffles may be evaluated by comparing the conversion obtained to the conversion calculated in an ideal unbaffled fluidized bed reactor operating at essentially plug flow, i.e., with no backmixing.

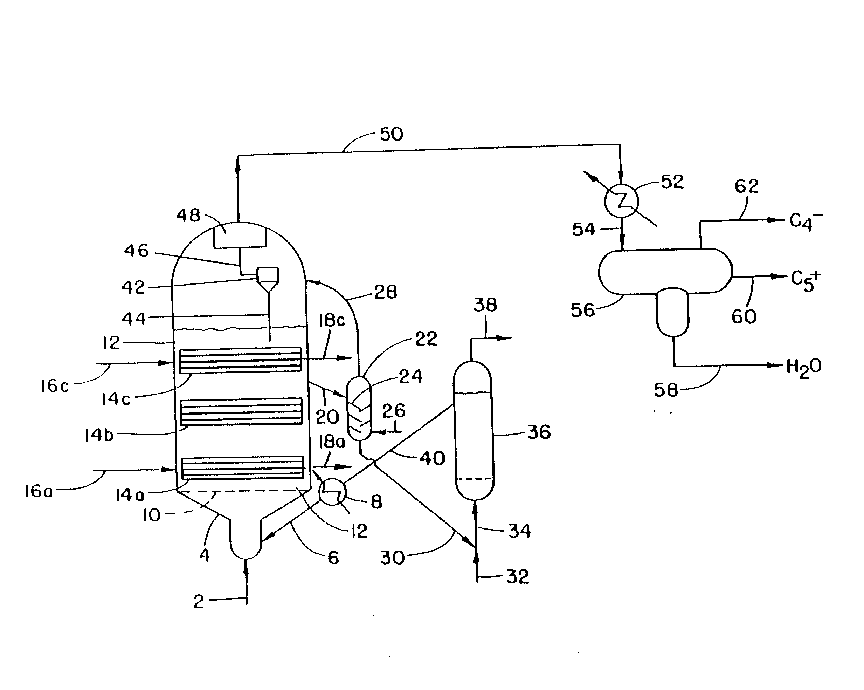

The process of the invention will now be described in greater detail by way of example only with reference to the accompanying drawing, which illustrates schematically the dense fluid bed reactor and ancillary equipment for carrying out the process. A methanol feed is used in this description, by way of example; any of the other feedstocks disclosed above containing, for example, oxygenated derivatives, for example, dimethyl ether, or oxygenates produced by Fisher-Tropsch synthesis, may be used to obtain advantageous results similar to those obtained when using methanol.

Referring to the drawing, a methanol feed containing up to 20 weight percent water is introduced preferably in a vaporous state, through line 2 into a bottom extended portion of reactor 4 where it is combined with freshly regenerated catalyst for line 6. The mixture of methanol feed and catalyst forms a suspension at a temperature from 200 to 425°C. The temperature may be regulated by employing a cooler 8 to adjust the temperature of the regenerated catalyst. The suspension of methanol reactant and zeolite catalyst then passes into the lower portion of reactor 4 through a distributor grid 10, positioned across the lower portion of the reactor 4 and enters the bottom of the dense fluid bed 12 of ZSM-5 catalyst. The catalyst density in the bed is from 32 to 640 Kg/m³. Positioned in the catalyst bed are rows of horizontal tubular baffles arranged in a series of bundles, illustrated as 14a, 14b and 14c. The individual tubular baffles have an external diameter from 25 to 75 mm. The rows within each bundle are staggered to provide a triangular or square pitch from 50 to 200 mm. The vertical distance between adjacent bundles, for example, between bundle 14a and bundle 14b is from 0.75 to 3 m, preferably from 0.9 to 1.8 m. Sufficient bundles of tubular baffles having the above configuration and spacing are located within the dense fluidized bed to provide a conversion efficiency from 65 to 85%. Since the horizontal bundles of tubular baffles are conveniently formed from heat exchanger tubes, a number of bundles are adapted for indirect heat exchange service to remove the exothermic heat of reaction that is released during the alcohol conversion. In some cases not all the bundles of tubular baffles will be required for heat exchanger service. Thus, bundle 14b is not serving as a heat exchanger while bundles 14a and 14c have been adapted to this service. Water is provided to these bundles through lines 16a and 16c so as to generate steam which passes from the bundles through lines 18a and 18c, respectively, for use as process steam.

The vaporous feed passes up through the dense fluidized bed 12 of ZSM-5 type zeolite catalyst under conversion conditions effective to provide a conversion of at least 95%. Conversions of 99.5% and above can be achieved. Useful conversion conditions include a WHSV from 0.5 to 20, a bed residence time from 3 to 30 seconds and a bed temperature from 340 to 430°C.

During the conversion, coke is formed on the catalyst. To maintain the activity of the catalyst, a portion of the catalyst is removed from reactor 4 through a line 20 for regeneration. The catalyst initially passes through a stripping vessel 22, provided with downwardly sloping baffles 24. Steam entering the bottom of the stripping vessel 22 through line 26 contacts the catalyst cascading down through the vessel 22 thereby stripping occluded hydrocarbons from the catalyst. The stripping steam and the stripped products pass from stripping vessel 22 through a line 28 and are discharged into the vapor space of reactor 4. The stripped catalyst flows from stripping vessel 22 through a line 30 and then it is passed, together with a molecular oxygen-containing gas supplied through a line 32, through a line 34 into a regenerator vessel 36 where a portion of the coke is oxidatively removed to provide a catalyst having the requisite activity. The resultant flue gas is removed from the regenerator through a line 38 and partially regenerated catalyst passes through a line 40 from where it is recycled through cooler 8 and line 2 to the lower section of reactor 4 for admixture with the fresh feed.

The reaction mixture passing up through the dense fluidized bed is converted to the desired degree as it passes through the horizontal rows of tubular baffles which are arranged in a staggered pattern to provide what amounts to a series of reaction zones. As the conversion proceeds the heat generated is removed by the generation of steam in heat exchanger bundles 14a and 14c. The reaction mixture passes from the upper level of the fluidized bed 12 into a vapor space in the upper end of reactor 4. Here the vaporous mixture passes through a cyclone separator 42 which removes entrained catalyst from the vapors and conducts it through dipleg 44 to catalyst bed 12. The vapors pass from the cyclone separator through a line 46 and into a plenum chamber 48. The vapor phase passing through the plenum chamber contains the hydrocarbon reaction products, water and traces of unreacted methanol. This vapor mixture passes from plenum chamber 48 through a line 50 to a cooler 52 where the water vapors and the normally liquid hydrocarbons are condensed. All of the reaction products then pass through a line 54 to a separator vessel 56 where the products are separated into three phases, an aqueous phase which is drawn off through line 58, a $C_5+$ hydrocarbon phase, i.e., the desired gasoline boiling range hydrocarbons, which is removed through line 60 and a $C_4-$ light hydrocarbon gas stream which is removed through line 62. The $C_5+$ stream is conventionally transferred to gasoline blending facilities where it is processed as required. The $C_4-$ stream may also be subject to further processing, i.e., alkylation to provide further components for gasoline blending.

The following example illustrates the invention.

Example

Methanol is passed to a dense fluidized bed reactor for converting methanol into gasoline boiling range hydrocarbons. The reactor contains a ZSM-5 zeolite catalyst in the form of a fine powder (0—150 μ) having a density of 0.5—1.5 g/cc. The operating temperature is 400—426°C, the reactor pressure is 70—345 kPa and the fluidized bed height is 4.5—10.5 m. The WHSV is 1—2 and the superficial gas velocity in the reactor is 0.45—0.75 m/sec. The fluidized bed reactor is operated (a) with vertical baffles in accordance with U.S. Patents 4,197,418 and 4,251,484 (b) without baffles and (c) with horizontal tubular baffles in accordance with the invention wherein the tubular baffles have a 50 mm O.D. on a 125 mm triangular pitch in bundles 1.2 m long and 1.2 m apart. The conversion efficiency, which is defined as the percentage of the conversion obtained in a given fluid bed reactor compared to the conversion obtained in a reactor operating at complete plug flow of the gas under comparable conditions, for each is as follows:

| | |
|---|---|
| Vertically baffled bed | 55—60% |
| Unbaffled bed | 55—60% |
| Horizontally baffled bed | 65—70% |

Claims

1. A process for converting a reactant comprising a $C_1$—$C_3$ monohydric alcohol, a related oxygenate, a Fischer-Tropsch oxygenate or a mixture of any two or more thereof, into a hydrocarbon product of higher boiling point which comprises passing a feed comprising the reactant alone or together with water upwardly through a dense fluidized bed of particles of a catalyst comprising a ZSM-5 type crystalline zeolite under exothermic conditions to convert at least 70% of the reactant into the hydrocarbon product, characterized in that the dense fluidized bed of catalyst particles contains a plurality of spaced, horizontally-disposed, tubular baffles in a plurality of spaced rows, the rows of baffles in the rows being so arranged in a staggered pattern as to provide between adjacent baffles a triangular or square configuration having a pitch of 50 to 200 mm.

2. A process according to claim 1, wherein the alcohol is methanol.

3. A process according to claim 1, wherein the related oxygenate is dimethyl ether.

4. A process according to any one of claims 1 to 3, wherein the tubular baffles have an external diameter from 25 to 75 mm.

5. A process according to any one of claims 1 to 4, wherein at least some of the baffles comprises heat exchanger tubes.

6. A process according to any one of claims 1 to 5, wherein the rows are arranged to form a plurality of bundles of baffles, each bundle having a vertical height of 0.3 to 1.4 m and being separated from the or each adjacent bundle by a vertical distance of 0.75 to 3 m.

7. A process according to claim 6, wherein the vertical distance is 0.9 to 1.8 m.

## Patentansprüche

1. Verfahren zur Umwandlung eines Reaktanten, der einen einwertigen $C_1$—$C_3$-Alkohol, eine verwandte Sauerstoffverbindung, eine Fischer-Tropsch-Sauerstoffverbindung oder eine Mischung von zwei oder mehreren davon umfaßt, in ein Kohlenwasserstoffproduct von höherem Siedpunkt, wobei das Verfahren das Leiten einer Zufuhr, die den Reaktanten allein oder zusammen mit Wasser umfaßt, aufwärts durch ein dichtes Wirbleschichtbett von Partikeln eines Katalysators, der einen kristallinen Zeolith vom ZSM-5-Typ umfaßt, unter exothermen Bedindungen umfaßt, um mindestens 70% des Reaktanten in das Kohlenwasserstoffprodukt umzuwandeln, dadurch gekennzeichnet, daß das dichte Wirbelschichtbett der Katalysatorpartikel eine Vielzahl von räumlich getrennten, waagerecht angeordneten rohrenförmigen Prallflächen in einer Vielzahl von räumlich getrennten Reihen enthält, wobei die Prallflächeneinreihen in den Reihen in einer versetzten Form so angeordnet sind, um zwischen benachbarten Prallflächen eine dreieckförmige oder quadratische Anordnung mit einem Abstand von 50 bis 200 mm zu schaffen.

2. Verfahren nach Anspruch 2, worin der Alkohol Methanol ist.

3. Verfahren nach Anspruch 1, worin die verwandte Sauerstoffverbindung Dimethyläther ist.

4. Verfahren nach einem der Ansprüche 1 bis 3, worin die rohrenförmigen Prallflächen einen äußeren Durchmesser von 25 bis 75 mm haben.

5. Verfahren nach einem der Ansprüche 1 bis 4, worin mindestens einige der Prallflächen Wärmeaustauscherrohre umfassen.

6. Verfahren nach einem der Ansprüche 1 bis 5, worin die Reihen angeordnet sind, um eine Vielzahl von Prallflächenbündeln zu bilden, wobei jedes Bündel eine Vertikalhöhe von 0,3 bis 1,4 m hat und von dem oder jedem benachbarten Bündel durch einen Vertikalabstand von 0,75 bis 3 m getrennt ist.

7. Verfahren nach Anspruch 6, worin der Vertikalabstand 0,9 bis 1,8 m beträgt.

## Revendications

1. Un procédé de conversion de réactifs comprenant un mono-alcool en $C_1$—$C_3$, un dérivé oxygéné qui lui est apparenté, un dérivé oxygéné de Fischer-Tropsch ou un mélange de deux ou plus deux d'entre eux, en un produit hydrocarboné de point d'ébullition plus élevé qui consiste à faire monter une charge comprenant le réactif seul ou en mélange avec de l'eau dans un lit fluidisé dense de particules d'un catalyseur comprenant une zéolite cristalline de type ZSM-5, dans des conditions exothermiques, pour convertir au moins 70% du réactif en produit hydrocarboné, caractérisé en ce que le lit fluidisé dense de particules de catalyseur contient une pluralité de chicanes tubulaires disposées horizontalement et espacés, formant une pluralité de rangées espacées, les rangées de chicanes étant disposées de façon étagée, de manière à former entre chicanes adjacentes une configuration triangulaire ou carrée présentant un pas de 50 à 200 mm.

2. Un procédé selon la revendication 1, où l'alcool est le méthanol.

3. Un procédé selon la revendication 1, où le dérivé oxygéné apparenté est l'éther diméthylique.

4. Un procédé selon l'une quelconque des revendications 1 à 3, dans lequel les chicanes tubulaires présentent un diamètre externe de 25 à 75 mm.

5. Un procédé selon l'une quelconque des revendications 1 à 4, dans lequel au moins une partie des chicanes comporte des tubes d'échange de chaleur.

6. Un procédé selon l'une quelconque des revendications 1 à 5, dans lequel les rangées sont disposées de façon à former une plurality de faisceaux de chicanes, chaque faisceau présentant une hauter verticale de 0,3 à 1,4 m et étant séparé du ou de chaque faisceau adjacent par une distance verticale de 0,75 à 3 m.

7. Un procédé selon la revendication 6, dans lequel la distance verticale est de 0,9 à 1,8 m.